# DEMANDE DE BREVET EUROPEEN

(11) **EP 3 892 308 A1**
(43) Date de publication de la demande: **13.10.2021**
(21) Numéro de dépôt: 21167067.4
(22) Date de dépôt: 06.04.2021
(51) Int. Cl.: A61L 2/22, A61L 9/14, B05B 7/08, A61L 9/12, B05B 7/00

(54) **DISPOSITIF DE PROJECTIONS DE PARTICULES DÉSINFECTANTES DANS L AIR**

(30) Priorité: 07.04.2020 FR 2003480
(71) Demandeur: Octopus Robots, 49300 Cholet (FR)
(72) Inventeur: VERGNE, Bertrand, 56400 Auray (FR); JOBARD, Mathieu, 49230 Sèvremoine (FR)
(74) Mandataire: Fédit-Loriot

(57) **Abrégé**

Dispositif (1) de projection de particules dans l'air comprenant un organe de ventilation (10) entraînant un flux d'air au travers d'une conduite d'air (11) s'étendant le long d'un axe principal d'extension (110) jusqu'à une bouche de sortie (122) présentant une section sensiblement circulaire, caractérisé en ce que le dispositif (1) comprend une pluralité de buses de projection de particules (14) installées de manière sensiblement circonférentielle autour de ladite bouche de sortie (122), chaque buse (14) étant orientée de sorte que son axe principal de projection (140) présente un angle inférieur à 90° par rapport à l'axe principal d'extension (110) de la conduite d'air (11) dans une direction concentrique.

## Description

L'invention concerne un dispositif de projections de particules désinfectantes dans l'air.

La désinfection par voie aérienne est une technique bien connue, consistant à pulvériser dans l'air des particules de produit désinfectant venant se déposer sur les zones à désinfecter.

L'invention concerne notamment la désinfection de grands espaces ouverts ou fermés, tels que des ateliers, des usines, des bureaux ou tout type de bâtiment, mais aussi des véhicules, notamment la cabine d'un aéronef.

Un problématique est d'assurer une projection diffuse, relativement homogène et à une distance contrôlée des particules dans l'air, afin d'assurer une désinfection optimale de la zone à désinfecter.

On connaît notamment le document WO2018/011501 décrivant un dispositif de nébulisation pour la désinfection par voie aérienne comprenant une tête ultrasonique faisant saillie d'un conduit de projection d'air et s'étendant perpendiculairement à la bouche de sortie de ce conduit au voisinage. Or un tel dispositif ne permet pas un contrôle optimal des conditions de projection des particules dans l'air.

Aussi, il existe le besoin d'un dispositif permettant un meilleur contrôle des conditions de projections des particules désinfectantes dans l'air.

On entend dans la présente demande par les termes de projection de particules ou pulvérisation, toute technique connue pour projeter un fluide liquide dans l'air, tel que l'atomisation, la nébulisation, la micro-nébulisation ou toute autre technique connue de l'art antérieur. L'invention pouvant être adaptée à tout type de buses de projection quelle que soit la taille des particules projetées dans l'air.

A cet effet, on propose un dispositif de projection de particules dans l'air comprenant un organe de ventilation entraînant un flux d'air au travers d'une conduite d'air s'étendant le long d'un axe principal d'extension jusqu'à une bouche de sortie présentant une section sensiblement circulaire, caractérisé en ce que le dispositif comprend une pluralité de buses de projection de particules installées de manière sensiblement circonférentielle autour de ladite bouche de sortie, chaque buse étant orientée de sorte que son axe principal de projection est parallèle ou présente un angle inférieur à 90° par rapport à l'axe principal d'extension de la conduite d'air dans une direction concentrique.

Ainsi, les buses sont agencées de sorte à assurer une projection optimale des particules dans le flux d'air, en permettant une distance de projection relativement élevée, par exemple allant à plus de deux mètres, tout en minimisant le risque de recombinaison des micro-gouttes de produit projeté pour en former de plus grosses,

En outre, un tel positionnement des buses permet de limiter la formation de gouttes perlantes en périphérie des buses.

En particulier, toutes les buses peuvent présenter une même valeur d'angle par rapport à l'axe principal d'extension, chacune orientée de manière sensiblement concentrique. Cependant l'invention n'est pas limitée à une même valeur d'angle pour toutes les buses, chaque buse pouvant avoir une valeur d'angle distincte par rapport aux autres.

Avantageusement et de manière non limitative, le dispositif comprend un organe de ventilation à hélices et un redresseur de flux installé dans ladite conduite d'air entre l'organe de ventilation à hélices et la bouche de sortie.

Ainsi on peut mettre en œuvre un ventilateur léger, peu coûteux et robuste tout en assurant que le flux d'air produit soit peu ou pas tourbillonnant. Ceci améliore ainsi la projection des particules désinfectantes dans le flux d'air.

Avantageusement et de manière non limitative, le redresseur de flux comprend une hélice fixe installée entre ledit organe de ventilation à hélice et ladite bouche de sortie. Ainsi, le redresseur de flux peut être peu coûteux à produire et relativement simple à installer dans le dispositif.

Selon une alternative avantageuse, le redresseur de flux comprend une pluralité d'ailettes solidaire de la paroi interne de ladite conduite d'air entre ledit organe de ventilation à hélice et ladite bouche de sortie. Une telle solution est relativement peu coûteuse et provoque une perte de charge relativement faible.

Avantageusement et de manière non limitative, ladite conduite d'air comprend une portion de sortie présentant une forme sensiblement tronconique. Ainsi, le flux d'air à la bouche de sortie est particulièrement relativement rapide et peu turbulent.

Selon une alternative avantageuse, ladite conduite d'air présente une forme sensiblement en cylindre droit, Ainsi, la conduite d'air est relativement peu coûteuse à fabriquer.

Avantageusement et de manière non limitative, le dispositif comprend un dispositif de déflection entre ledit dispositif redresseur de flux et ladite bouche de sortie. Ainsi, on peut contrôler la forme du flux d'air et sa vitesse en sortie du dispositif.

Avantageusement et de manière non limitative, le dispositif comprend au moins quatre buses, et par exemple dix buses, réparties régulièrement autour de ladite bouche de sortie. Ainsi, on peut assurer une projection d'une importante quantité relativement de produit désinfectant tout en assurant une qualité de projection optimale.

Avantageusement et de manière non limitative, lesdites buses comprennent des buses de nébulisation et/ou de micro ou nano nébulisation. Ainsi, on peut produire des particules relativement fines de produit désinfectant, ce qui assure une projection optimale du désinfectant dans l'air.

L'invention concerne aussi un ensemble mobile de désinfection d'une surface à désinfecter comprenant une base mobile motorisée dans laquelle sont installés un réservoir de liquide désinfectant et au moins un dispositif tel que décrit précédemment, pour lequel lesdites buses comprennent des moyens d'alimentation en liquide désinfectant conformés pour pomper ledit liquide dans ledit réservoir et pour les projeter en sortie desdites buses.

D'autres particularités et avantages de l'invention ressortiront à la lecture de la description faite ci-après de deux modes de réalisation particuliers de l'invention, donnés à titre indicatif mais non limitatifs, en référence aux dessins annexés sur lesquels :
[Fig. 1] est une vue en éclaté d'un premier mode de réalisation du dispositif ;
[Fig. 2] est une vue en perspective du dispositif selon la figure 1 ;
[Fig. 3] est une vue de face, depuis la bouche de sortie, du dispositif selon la figure 1 ;
[Fig. 4] est une vue en éclaté d'un deuxième mode de réalisation du dispositif ;
[Fig. 5] est une vue en perspective du dispositif selon la figure 4 ;
[Fig. 6] est une vue de face, depuis la bouche de sortie, du dispositif selon la figure 4 ; et
[Fig. 7] est une vue schématique d'une base mobile comprenant des dispositifs selon l'invention.

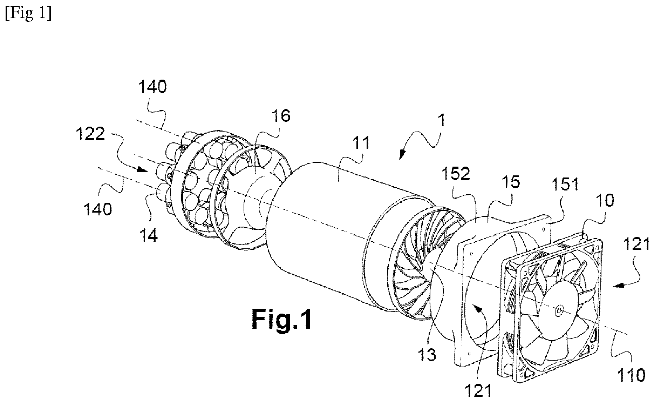

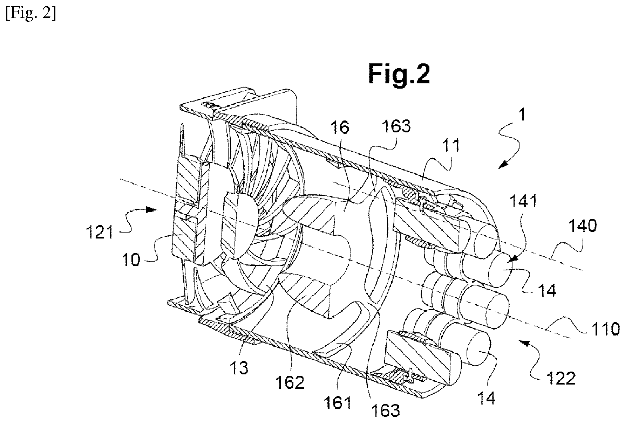

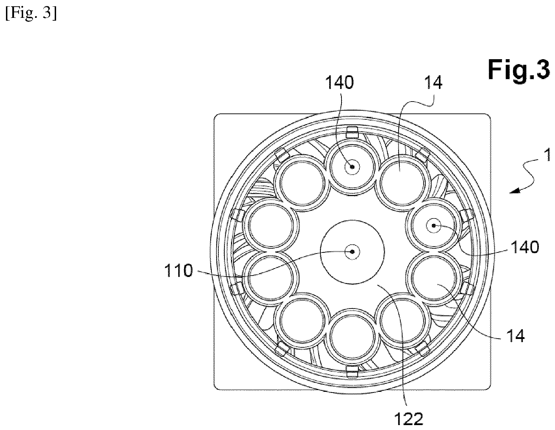

Selon un premier mode de réalisation de l'invention en référence aux figures 1 à 3, un dispositif de projection 1 de particules désinfectantes dans l'air comprend un organe de ventilation 10 et une conduite d'air 11 guidant le flux d'air depuis une extrémité d'entrée 121 jusqu'à une bouche de sortie 122, le long d'un axe principal d'extension 110.

La conduite d'air 11 peut être fait de toute matériau adapté, tel que des métaux ou des matériaux plastiques.

L'organe de ventilation 10 est dans ce mode de réalisation un ventilateur à hélice 10, aussi appelé plus simplement ventilateur 10, qui est fixé à la conduite d'air 11 par une bride 15.

La bride 15 comprend d'une part une platine de fixation 151 apte à fixer le ventilateur 10 et d'autre part une section tubulaire 152 conformée pour s'engager autour de la conduite d'air 11 de sorte à former un joint de fixation.

Selon une mise en œuvre alternative, la section tubulaire 152 peut être conformée pour s'engager dans la conduite d'air 11, venant en butée par exemple jusqu'à la platine de fixation 151.

Le ventilateur électrique 10 constitue un moyen simple et peu coûteux pour propulser de l'air dans une conduite d'air 11. En particulier dans un contexte où le dispositif 1 est embarqué sur une base mobile automobile, un tel ventilateur électrique 10 est peu énergivore. Toutefois un inconvénient d'une telle solution est que le ventilateur électrique 10 produit un flux d'air tourbillonnant.

Aussi, afin de contrer l'effet de ce flux tourbillonnant, un redresseur de flux 13 est installé entre le ventilateur 10 et la bouche de sortie 122.

Ce redresseur de flux 13 comprend une hélice fixe, connue de l'art antérieur, donc les pales sont orientées de sorte à contrer le tourbillon d'air produit par l'hélice du ventilateur 10 ; le flux d'air en sortie de la bouche de sortie 122 étant alors faiblement tourbillonnant ou non tourbillonnant.

Selon une alternative le redresseur de flux 13 peut aussi être fait d'une pluralité d'ailettes solidaires des parois internes de la conduite d'air 11, et installées cir-conférentiellement de manière régulière. Tout autre dispositif de redressage d'un flux d'air tourbillonnant pouvant être mis en œuvre dans l'invention.

Toutefois l'invention n'est pas limitée à la seule utilisation d'un ventilateur à hélice 10, mais peut impliquer tout autre dispositif de ventilation adapté pour propulser le flux d'air au travers de la conduite d'air 11.

La bouche de sortie 122 de la conduite d'air 11 présente une forme sensiblement circulaire.

La bouche de sortie 122 est en particulier centrée autour de l'axe principal d'extension 110 de la conduite d'air 11, de sorte à assurer une projection optimale de l'air en sortie de la conduite 11.

On entend par sensiblement circulaire toute forme elliptique, cercle ou ovale notamment.

Une pluralité de buses de projection 14 de particules désinfectantes, sont installées de sorte à projeter les particules au voisinage de la bouche de sortie 122.

Dans ce mode de réalisation, le dispositif comprend 10 buses installées et réparties de manière régulière de manière sensiblement circulaire tout autour de la bouche de sortie 122.

Comme exposé précédemment, on entend par buse de projection 14 tout type de buse connu de l'art antérieur, tel que des buses de micronisation, de pulvérisation, d'atomisation, de nébulisation, de micro-nébulisation, par exemple basée sur des organes tels que des transducteurs piézoélectriques, notamment grillagés, ou selon toute autre technique connue de l'art antérieur.

L'invention n'est pas limitée à un nombre fixe de buses, et on privilégiera un nombre de buses allant de 1 à 20 buses, et en particulier de 4 à 12 buses.

Les buses 14 sont installées de sorte à projeter des particules de produit désinfectant au contact du flux d'air projeté en sortie de la bouche de sortie 112.

A cet effet, les buses 14 présentent une extrémité de projection 141 orientée de sorte à projeter selon un axe de projection principale 140 sensiblement parallèle ou formant un angle aigu avec l'axe d'extension principal 110.

En effet, on cherche à orienter les buses de manière sensiblement tangente à la paroi interne de la conduite d'air 11 au voisinage de la bouche de sortie 122, tout en formant un angle faiblement rentrant vers le centre de la bouche de sortie 122.

En particulier, dans ce mode de réalisation on privilégiera un tel angle faiblement rentrant compris entre 0° et 20°, préférentiellement entre 1° et 10°, et orienté selon au moins une direction de manière convergente vers le centre de la bouche de sortie 122.

L'angle rentrant des buses 14 peut être déterminé par l'homme du métier, notamment en fonction de la puissance du ventilateur et en fonction de la concentration de pulvérisation souhaitée.

Les buses sont dans ce mode de réalisation des buses de nébulisation ou de micro-nébulisation, ou tout autre type de buse apte à projeter dans l'air des particules de taille comprise entre 100 nm et 200 µm, en particulier entre 5 µm et 20 µm, par exemple 12 µm.

L'homme du métier sait qu'une telle buse, quel qu'en soit le modèle, doit notamment être alimentée électriquement et comprendre un système d'alimentation en fluide désinfectant, tel qu'une pompe, ce qui n'est pas l'objet de l'invention et n'est par conséquent pas décrit.

Une telle configuration de buses réparties autour de la bouche de sortie de laquelle est propulsé un flux d'air permet d'obtenir une propagation des particules désinfectantes de manière peu turbulente, et en assurant que ces particules ne se regroupent pas, formant des gouttelettes plus lourdes et peu volatiles, ce qui doit être évité.

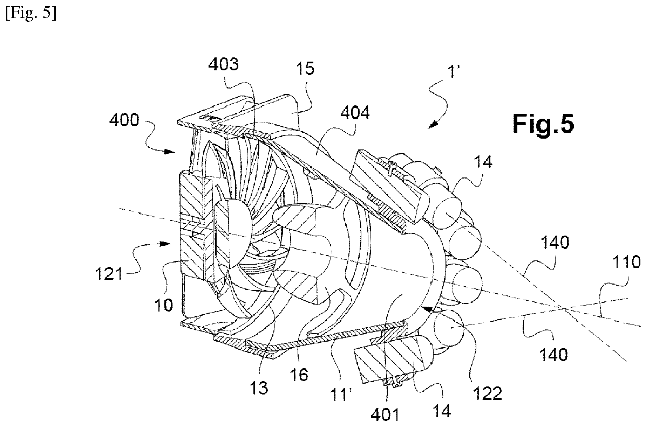

Les buses peuvent être installées, selon des alternatives de mises en œuvre de l'invention, soit à l'extérieur de la conduite d'air, tel qu'il est représenté figure 5 pour le deuxième mode de réalisation, soit à l'intérieur de la conduite d'air 11, tel que représenté pour ce premier mode de réalisation.

L'installation des buses à l'intérieur de la conduite d'air 11 présente l'avantage d'obtenir un dispositif plus compact, mais nécessite des adaptations pour assurer l'alimentation électrique et en fluide des buses, sans encombrer de manière excessive l'intérieur de la conduite d'air 11. A contrario l'installation des buses à l'extérieur de la conduite d'air 11 impose un encombrement sensiblement plus important, mais simplifie la problématique d'alimentation en fluide et électrique des buses.

Selon une mise en œuvre alternative de ce mode de réalisation, un dispositif de déflexion 16 du flux d'air peut être installé dans la conduite d'air 11 entre le dispositif de redresseur 13 et la bouche de sortie 122.

Un tel dispositif de déflexion 16 permet de moduler la forme de l'écoulement de l'air dans la conduite d'air 11.

Dans ce mode de réalisation le dispositif de déflexion 16, aussi appelé déflecteur, est fait d'une pièce moulée, comprenant une section circulaire 161, maintenu en force radialement contre les parois internes de la conduite d'air 11 et une portion centrale 162 présentant une forme de toroïde de section droite profilée en regard du ventilateur 10, la portion centrale 162 étant fixée à la section circulaire 161 par une pluralité de tige radiales de fixation 163.

De tels dispositifs de déflexion peuvent être librement adaptés par l'homme du métier de sorte à conformer le flux d'air en sortie de la conduite d'air 11 en fonction du besoin de type de pulvérisation, par exemple de manière lointaine et fortement directionnelle ou au contrairement de manière relativement proche et diffuse. Ces déflecteurs 16 peuvent en outre de produire une accélération du flux d'air en sortie du dispositif 1.

On peut prévoir selon un autre mode de réalisation particulier, d'installer le dispositif de déflexion 16 de manière mobile en translation le long de l'axe principale d'extension 110, de sorte à permettre une modulation de la forme du flux d'air et sa vitesse de manière dynamique lors de l'utilisation du dispositif.

Ainsi, le dispositif de déflexion 16 peut être entrainé en translation par un moteur électrique ou tout autre dispositif tel qu'un vérin, un vérin à vis ou tout autre solution adaptée pour translater en fonction d'une commande électrique le déflecteur 16 dans la conduite d'air 11.

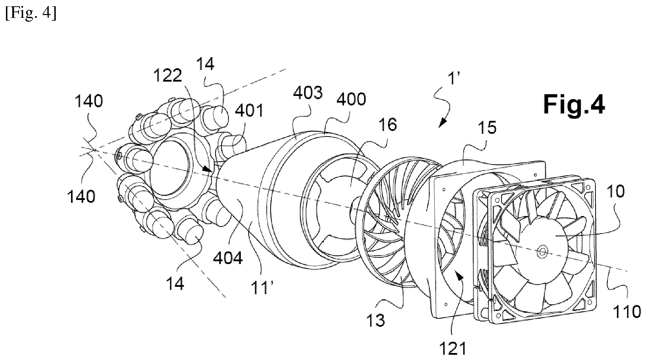

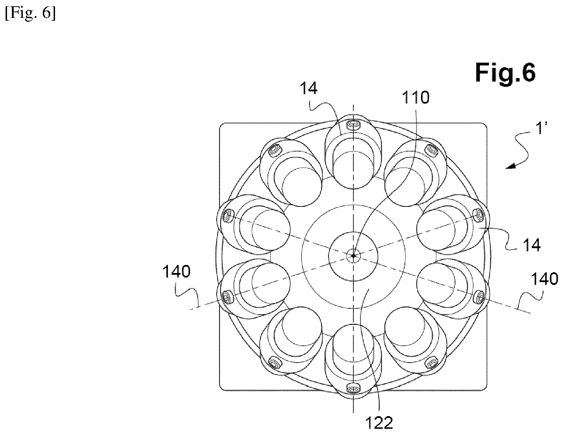

Selon un deuxième mode de réalisation, en référence aux figures 4 à 6, le dispositif 1' diffère de celui du premier mode de réalisation en ce que la conduite d'air 11' présente une portion d'entrée cylindrique 403 et une portion de sortie tronconique 404.

La conduite d'air 11' comprend alors une section large 400 contre laquelle est montée la bride 15, et une section étroite 401 formant bouche de sortie 122.

De par la forme relativement étroite de la bouche de sortie 122, l'installation des buses 14 doit alors être effectuée depuis l'extérieur de la conduite d'air 11'.

Les buses 14 sont alors orientées, comme exposé pour le premier mode de réalisation, selon un angle rentrant par rapport à l'axe principale d'extension 110 du tube.

En particulier les buses 14 sont orientées sensiblement parallèlement à la génératrice de la section tronconique de la portion de sortie tronconique 404.

Toutefois un angle supplémentaire peut être ajouté de sorte que les buses 14 présentent un angle rentrant, orienté vers le centre de la bouche de sortie 122 En particulier un angle aigu allant de 1° à 10°. Toutefois les buses peuvent aussi être maintenues strictement parallèles à la génératrice de la section tronconique 404.

Toutefois la forme de la conduite d'air 11, 11' n'est pas limitée à un cylindre de révolution ou à la forme décrite dans le deuxième mode de réalisation présentant une section tronconique 404. En particulier la conduite d'air peut présenter toute forme connue notamment dans le domaine des tuyères, par exemple avec une portion de sortie en forme de jupe.

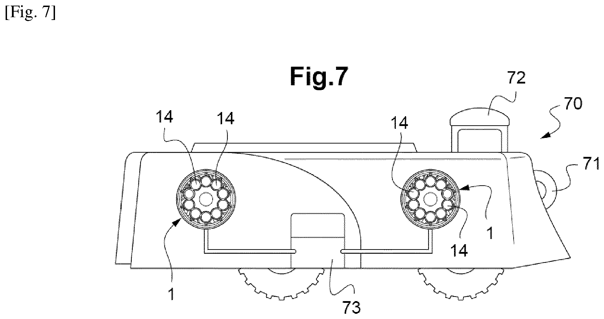

L'invention concerne aussi une base mobile motorisée 70, représentée figure 7, sur laquelle est installée un ou plusieurs dispositifs 1 selon l'invention. La figure 7 est une vue schématique qui représente des dispositifs selon le premier mode de réalisation. Toutefois la base mobile 70 peut embarquer toute variation et/ou combinaison de dispositifs selon l'invention, notamment des premiers et deuxièmes modes de réalisation.

La base mobile 70 peut être radioguidée ou autonome ; dans ce dernier cas, représenté figure 7, la base mobile comprend des moyens de navigation autonome 71, 72, tel qu'un radar 72 et un lidar 71, permettant de calculer un itinéraire de pulvérisation dans une zone à désinfecter et de parcourir de manière autonome cette zone à désinfecter. Toutefois la base mobile 70 peut comprendre tout autre dispositif de localisation dans l'espace et de calcul de trajectoire, sans être limité à ce qui est représenté figure 7.

La base mobile 70 est de manière préférée auto-alimentée et embarque à cette fin au moins une batterie d'accumulateurs électriques, et de sorte à alimenter électriquement la motorisation de la base mobile ainsi que les buses 14 et le ventilateur 10 du dispositif selon l'invention,

La base mobile 70 comprend aussi un réservoir 73 de produit désinfectant dans lequel les buses 14 des dispositifs 1 pompent, par des moyens non représentés mais connus de l'homme du métier, le produit désinfectant afin de le projeter dans l'air.

## Revendications

1. Dispositif (1, 1') de projection de particules dans l'air comprenant un organe de ventilation (10) entraînant un flux d'air au travers d'une conduite d'air (11, 11') s'étendant le long d'un axe principal d'extension (110) jusqu'à une bouche de sortie (122) présentant une section sensiblement circulaire, **caractérisé en ce que** le dispositif (1, 1') comprend une pluralité de buses (14) de projection de particules installées de manière sensiblement circonférentielle autour de ladite bouche de sortie (122), chaque buse (14) étant orientée de sorte que son axe principal de projection (140) est parallèle ou présente un angle inférieur à 90° par rapport à l'axe principal d'extension (110) de la conduite d'air (11, 11') dans une direction concentrique.

2. Dispositif selon la revendication 1 **caractérisé en ce que** le dispositif (1, 1') comprend un organe de ventilation à hélices (10) et un redresseur de flux (13) installé dans ladite conduite d'air entre l'organe de ventilation à hélices (10) et la bouche de sortie (122).

3. Dispositif (1, 1') selon la revendication 2, **caractérisé en ce que** le redresseur de flux (13) comprend une hélice fixe (131) installée entre ledit organe de ventilation à hélice (10) et ladite bouche de sortie (12).

4. Dispositif (1, 1') selon la revendication 2, **caractérisé en ce que** le redresseur de flux (13) comprend une pluralité d'ailettes solidaires de la paroi interne de ladite conduite d'air (11) entre ledit organe de ventilation à hélice (10) et ladite bouche de sortie (122).

5. Dispositif (1') selon l'une quelconque des revendications 1 à 4, **caractérisé en ce que** ladite conduite d'air (11') comprend une portion de sortie (404) présentant une forme sensiblement tronconique.

6. Dispositif (1) selon l'une quelconque des revendications 1 à 4, **caractérisé en ce que** ladite conduite d'air (11) présente une forme sensiblement en cylindre droit.

7. Dispositif (1, 1') selon l'une quelconque des revendications 1 à 6, **caractérisé en ce qu'**il comprend un dispositif de déflection (16) entre ledit dispositif redresseur de flux (13) et ladite bouche de sortie (122).

8. Dispositif (1, 1') selon l'une quelconque des revendications 1 à 7, **caractérisé en ce qu'**il comprend au moins quatre buses (14) réparties régulièrement autour de ladite bouche de sortie (122).

9. Dispositif selon l'une quelconque des revendications 1 à 8, **caractérisé en ce que** lesdites buses (14) comprennent des buses de nébulisation et/ou de micro-nébulisation.

10. Ensemble mobile de désinfection d'une surface à désinfecter comprenant une base mobile motorisée (70) dans laquelle sont installés un réservoir (73) de liquide désinfectant et au moins un dispositif (1, 1') selon l'une quelconque des revendications 1 à 9, pour lequel lesdites buses (14) comprennent des moyens d'alimentation en liquide désinfectant conformés pour pomper ledit liquide dans ledit réservoir (73) et pour les projeter en sortie desdites buses (14).
